Europäisches Patentamt

European Patent Office

Office européen des brevets

(19)

(11) Numéro de publication : **0 390 624 B1**

(12)                           **FASCICULE DE BREVET EUROPEEN**

(45) Date de publication du fascicule du brevet :
**08.06.94 Bulletin 94/23**

(51) Int. Cl.$^5$ : **C12P 7/26**

(21) Numéro de dépôt : **90400518.8**

(22) Date de dépôt : **26.02.90**

(54) **Procédé microbiologique d'obtention de méthylcétones.**

(30) Priorité : **27.02.89 FR 8902524**

(43) Date de publication de la demande :
**03.10.90 Bulletin 90/40**

(45) Mention de la délivrance du brevet :
**08.06.94 Bulletin 94/23**

(84) Etats contractants désignés :
**BE CH DE DK FR GB IT LI LU NL**

(56) Documents cités :
**EP-A- 0 054 987**
**EP-A- 0 068 594**
**FR-A- 2 617 501**
**ENZYME MICROB. TECHNOL., vol. 11, février 1989, Butterworth Publishers; C. LARROCHE et al., pp. 106-112**

(73) Titulaire : **ELF SANOFI**
**32-34, rue Marbeuf**
**F-75008 Paris (FR)**

(72) Inventeur : **Creuly, Catherine**
**Résidence du Puy d'Anzelle**
**F 63370 Lempdes (FR)**
Inventeur : **Gros, Jean-Bernard**
**7, Rue des Galoubiers**
**F-63400 Chamalieres (FR)**
Inventeur : **Larroche, Christian**
**34, Avenue Léon Blum**
**F-6300 Clermont-Ferrand (FR)**

(74) Mandataire : **Gillard, Marie-Louise et al**
**Cabinet Beau de Loménie**
**158, rue de l'Université**
**F-75340 Paris Cédex 07 (FR)**

EP 0 390 624 B1

## Description

La présente invention concerne un procédé d'obtention de méthylcétones par incubation d'acides carboxyliques avec des spores de champignons en milieu organique.

Parmi les molécules aromatiques caractéristiques de la saveur des fromages à inclusions de "bleu", se trouvent des méthylcétones, et notamment l'heptanone-2 et la nonanone-2, qui résultent dans la nature d'une β-oxydation suivie d'une décarboxylation des acides gras à un carbone de plus par des enzymes présentes dans les spores et le mycélium du champignon Penicillium roquefortii. On peut se référer, par exemple, à la revue de John E. Kinsella et Dong H. Hwang, publiée dans Critical Reviews in Food Science and Nutrition 8 (2) p. 191-228 (1976), qui fait la synthèse des nombreux travaux publiés sur ce sujet et cite un certain nombre d'études de fermentation in vitro.

Ces composés peuvent être obtenus par action, sur les matières grasses du lait, de ces enzymes présentes, à des degrés divers, dans différents champignons filamenteux de la division III regroupant les Amastigomycota et en particulier dans les Deuteromycotina, selon la classification établie par Botton et Coll dans "Moisissures utiles et nuisibles" Ed Masson - Paris FR (1985) dont Penicillium roquefortii Penicillium caseicolum, Aspergillus oryzae, Aspergillus niger et Trichoderma koningii. Ces cétones sont utilisées pour renforcer le goût des fromages encore jeunes ou pour aromatiser au "bleu" des produits alimentaires divers, tels que sauces, biscuits, laitages ; ces produits sont très appréciés des consommateurs et il était souhaitable, pour permettre leur large diffusion, de diminuer le coût de leur fabrication. Pour cela, il fallait notamment améliorer la bioconversion des acides gras en méthylcétones aromatisantes, que ce soit au stade de la production des spores, ou lors de la production des composés aromatisants sous l'influence des spores. Diverses études ont été effectuées dans cette optique et C. Larroche et al. ont décrit un procédé amélioré de production des spores de Penicillium roquefortii par fermentation en milieu solide, sur grains de sarrasin, dans Appl. Microbiol. Biotechnol. 28 85-92 (1988) et rapporté leur étude de l'influence de la souche de Penicillium et de divers activateurs sur la production de l'heptanone-2 à partir de l'acide octanoïque en milieu aqueux, dans J. of Ind. Microbiology 3 p. 1-8 (1988), et avec des spores immobilisées dans Enzyme Microbiotechnology 11 (2) p. 106-112 (1989).

On a maintenant trouvé que l'on pouvait augmenter très sensiblement la concentration en acide gras dans le milieu, la productivité en méthylcétones des spores, et diminuer les pertes en méthylcétones volatiles en effectuant la bioconversion en milieu organique, et cela aussi bien avec des spores immobilisées qu'avec des spores libres.

Les exemples de bioconversion en milieu organique ne sont pas nombreux malgré leurs avantages potentiels, de nombreux biocatalyseurs étant inactivés ou dénaturés par les solvants organiques usuels ; il est donc surprenant de constater que les spores de Penicillium roquefortii et des autres champignons filamenteux précédemment cités effectuent la bioconversion des acides gras en méthylcétones dans les milieux organiques de l'invention avec des résultats supérieurs à ceux obtenus en milieu purement aqueux.

Ainsi, la présente invention concerne un procédé d'obtention de méthylcétones aliphatiques en $C_5$ à $C_{10}$ par conversion d'un acide gras ou d'un mélange d'acides gras convenable en $C_6$ à $C_{11}$, en milieu organique aérobie, sous l'action de spores de champignons, libres ou immobilisées dans une matrice poreuse, par exemple dans une matrice d'alginate ou de gélatine réticulés.

Le solvant organique utilisé comme milieu réactionnel doit évidemment être inerte mais aussi n'avoir qu'une faible polarité ; on a ainsi constaté que, pour l'acide octanoïque, la réaction n'avait pas lieu dans l'éther éthylique ou le toluène et que, dans l'hexane ou le méthylcyclohexane, les résultats étaient proches de ceux obtenus en milieu aqueux, tandis que les hydrocarbures aliphatiques en $C_8$ à $C_{20}$, c'est-à-dire les hydrocarbures liquides paraffiniques, isoparaffiniques et cycloparaffiniques, purs ou en mélanges, permettent d'obtenir des rendements de bioconversion supérieurs à ceux des milieux aqueux. Aussi, pour la mise en oeuvre du procédé de l'invention, on utilise, comme milieu réactionnel, un solvant pratiquement apolaire, c'est-à-dire dont le coefficient de partage P entre l'eau et l'octane est élevé. On considère ici que P est élevé si log P, déterminé par la méthode de R.F. Rekker décrite dans Eur. J. Med. Chem. 14 479 (1979), est supérieur à 4 et de préférence compris entre 7 et 9.

Le solvant intervenant dans le procédé de l'invention est donc avantageusement choisi parmi les hydrocarbures aliphatiques en $C_8$ à $C_{20}$ et leurs mélanges, qui présentent les caractéristiques énoncées ci-dessus. Il peut notamment consister en un mélange d'hydrocarbures paraffiniques en $C_{13}$ à $C_{16}$. Ledit solvant, comme explicité ci-après, peut contenir jusqu'à 20% d'eau.

Les spores utilisées dans le procédé de l'invention sont obtenues selon des procédés classiques de fermentation en phase solide ou liquide. Après une fermentation du champignon en milieu solide, on peut utiliser comme biocatalyseur le milieu complet de fermentation, par exemple lorsque la fermentation a eu lieu sur graines de sarrasin ; on peut aussi séparer les spores externes du milieu, de façon classique, en mettant le milieu en fin de fermentation en suspension dans de l'eau en présence d'un tensioactif tel que le Tween® 80 avant de séparer le liquide contenant les spores des solides par filtration, après quoi les spores pourront être isolées si désiré par centrifugation.

Les spores isolées peuvent être introduites en l'état dans le milieu de bioconversion ou après immobilisation dans une matrice constituée de billes d'alginate, consolidées après l'inclusion par action de la polyéthylèneimine et du glutaraldéhyde, comme décrit par S. Birnbaum dans Biotechnol. Lett. 3 p. 393-400 (1982), ou par enrobage avec un polymère, tel qu'un copolymère acrylique, selon le procédé décrit par W. Hartmeier et A. Heinrichs dans Biotechnol. Lett. 8 p. 567-572 (1986) pour l'immobilisation de mélanges de cellules microbiennes et d'enzymes ; elles peuvent aussi être immobilisées dans des microbilles de gélatine réticulée avec un polysaccharide oxydé, comme décrit dans la demande de brevet EP-A-308 330 ou par toute autre méthode connue de l'homme du métier.

Lorsque les spores ne sont pas utilisées rapidement, elles sont conservées congelées vers -15°C.

Il est évident que le milieu de bioconversion n'est pas anhydre, car une certaine quantité d'eau est introduite avec les spores, qu'elles aient été isolées ou immobilisées, et on a constaté que, si le volume d'eau ne représente que quelques % du volume du milieu organique selon l'invention, les résultats de la bioconversion sont nettement supérieurs à ceux obtenus en milieu purement aqueux. Néanmoins, comme il est préférable qu'une certaine quantité d'eau soit présente pendant toute la durée de la bioconversion et pour éviter la déshydratation qui serait due à l'entraînement de l'eau par le courant d'air nécessaire à ces réactions aérobies, on peut commencer la bioconversion avec un volume d'eau qui représente jusqu'à 20 % du volume du solvant organique ; si l'on n'introduit dans le milieu au départ que 5 % d'eau environ, on préfère alors compenser les pertes en introduisant de l'air chargé d'humidité.

On introduit l'acide à transformer dans le milieu réactionnel contenant les spores par étapes successives ou en continu : les concentrations optimales d'acide dans le milieu réactionnel dépendent de la nature de l'acide mis en jeu, de celle du solvant et du champignon, mais sont toujours nettement supérieures à celles admissibles lorsque le procédé est mis en oeuvre en milieu purement aqueux, et c'est un autre avantage de l'invention.

Ainsi, dans le cas de l'acide octanoïque et d'un solvant isoparaffinique de log P voisin de 7, la bioconversion n'est pas inhibée par une concentration d'acide dans le milieu aussi élevée que 300 mM par litre de solvant et pour environ $10^{11}$ spores de Penicillium roquefortii immobilisées dans de l'alginate réticulé, en suspension dans un solvant paraffinique en $C_{13}$ à $C_{16}$, le rendement est voisin de 80 % pour une concentration ajustée à 100 mM.

La température de la bioconversion dépend du champignon utilisé et l'homme de métier pourra la fixer après quelques essais préalables ; elle est, en général, inférieure à 30°C et, mieux, comprise entre 25°C et 30°C.

Dans ce qui suit, des exemples de mise en oeuvre du procédé de l'invention sont décrits.

Exemple 1 :

a) Obtention des spores

On inocule 300 g de grains de sarrasin, cuits au bainmarie à 100°C pendant 30 min dans de l'eau contenant 350 mg/l de chloramphénicol puis stérilisés à l'autoclave 20 min à 120°C après égouttage, à raison de $10^5$ à $10^6$ spores, provenant de la souche de Penicillium roquefortii ATCC 64 383, par gramme de matière sèche.

Ces grains sont mis dans des fermenteurs aérés préalablement stérilisés et on obtient en 12 à 15 jours environ $5 \times 10^9$ spores/g de matière sèche. Les spores externes aux grains sont alors extraites par agitation à température ambiante dans une solution aqueuse de Tween $80^R$ à 0,05 %, à raison de 5 à 10 ml de solution par gramme de milieu de fermentation, et les grains sont séparés par filtration. Une solution concentrée de spores libres est obtenue après décantation ou, pour obtenir un milieu plus concentré, après centrifugation de ce filtrat.

Pour conserver plus de 24 heures les spores, on peut les congeler à une température inférieure à -15°C soit dans le milieu aqueux d'isolement, soit dans leur milieu de fermentation, auquel cas il faudra réhydrater les spores par maintien en suspension dans l'eau à 5°C pendant 10 h au moins avant leur utilisation.

b) Immobilisation des spores dans l'alginate

On introduit $8 \times 10^{10}$ spores isolées en suspension dans 10 ml d'eau dans 120 ml d'une solution à 2 % (p/v) d'alginate de sodium, préalablement homogénéisée par agitation violente, puis désaérée sous vide.

Le mélange est ensuite pompé à l'aide d'une pompe péristaltique pour passer à travers des aiguilles de 0,6 mm de diamètre, ce qui permet la formation de gouttelettes qui sont durcies en tombant dans 200 ml d'une émulsion constituée d'une solution aqueuse d'un mélange de $CaCl_2$ et d'un copolymère acrylate/méthacrylate de masse moléculaire voisine de 150 000, tel que l'Eudragit RL100[R] commercialisé par Rohm Pharma (RFA) ; l'émulsion a été préparée par introduction à 18°C dans un volume de solution aqueuse de $CaCl_2$ 0,1 M, d'un volume d'une suspension d'Eudragit à 1 % (p/v) dans de l'eau distillée, préalablement chauffée pendant 20 min à 121°C.

La suspension de billes est agitée 30 min après la fin de l'addition des gouttelettes d'alginate puis abandonnée 15 h à 5°C ; le liquide surnageant est alors éliminé et les billes, dont le diamètre est de 3 mm environ, sont rincées deux fois avec de l'eau distillée stérile contenant 350 mg/l de chloramphénicol. On obtient ainsi 130 g de billes qui contiennent environ $8 \times 10^{10}$ spores.

c) Bioconversion

On introduit les billes humides préparées selon b) dans 400 ml d'un solvant isoparaffinique, commercialisé par Total Solvants (France) sous la référence IP 230 OS, constitué d'un mélange d'hydrocarbures saturés en $C_{13}$ à $C_{16}$, de points d'ébullition supérieurs à 230°C, de viscosité mesurée selon la norme NF T 60-100 à 20°C égale à 4,10 mm²/s. Le réacteur de 750 ml est agité par une turbine à pales tournant à 600 tr/min, et on introduit dans le milieu de l'air à raison de 1,2 l par heure ; l'ensemble est maintenu à 27°C par une circulation de liquide dans la double enveloppe du réacteur.

On introduit 7 g (100 mM) d'acide octanoïque au début de la bioconversion, puis, toutes les 12 heures environ, on réajuste la concentration en acide octanoïque dans le réacteur à sa valeur de départ (100 mM), par addition de la quantité nécessaire d'acide. Au bout de 212 h, la quantité d'acide transformée est de 111 g et on isole par distillation à 79°C sous 9 kPa à partir du solvant, après filtration du catalyseur, 70 g d'heptanone-2. Une partie de la cétone formée a été entraînée par la circulation d'air et, si un piège est installé à la sortie du réacteur, on récupère encore environ 11 g de cétone.

Exemple 2 :

On prépare le catalyseur comme à l'exemple 1, et on introduit 50 g de billes contenant $2 \times 10^8$ spores par gramme dans 150 ml du même solvant que précédemment dans une fiole d'Erlenmeyer agitée maintenue à 27°C, avec seulement une aération de surface. 28 heures après l'addition de 2,4 g d'acide octanoïque, on constate que tout l'acide a été transformé ; le rendement en heptanone-2 isolée est de 80 %.

Exemple 3 :

On prépare le catalyseur comme à l'exemple 1, et on introduit 50 g de billes contenant $2 \times 10^8$ spores par gramme dans 150 ml d'hexadécane dans un réacteur agité maintenu à 27°C avec seulement une aération de surface. On introduit 2,4 g (100 mM) d'acide octanoïque au début de la bioconversion, puis, toutes les 24 heures environ, on réajuste à 100 mM la concentration en acide octanoïque dans le réacteur.

Au bout de 160 h, la quantité d'acide transformée est de 9,6 g et on isole par distillation du solvant du milieu réactionnel 6,5 g d'heptanone-2.

Exemple 4 :

En opérant dans les mêmes conditions qu'à l'exemple 1 avec de l'acide caprique en $C_{10}$, à la concentration initiale de 100 mM, le rendement moyen de conversion en nonanone est de 70 % et la productivité - c'est-à-dire la concentration d'acide transformé - est de 0,71 mmole par heure.

Exemple 5 :

En opérant dans les mêmes conditions qu'à l'exemple 1, mais avec de l'acide caproïque en $C_6$, à la concentration initiale de 50 mM, le rendement moyen de conversion est de 70 % et on obtient 8 g/l de pentanone-2 en 5 jours.

Exemple 6 :

On introduit 180 g de grains de sarrasin sporulés, obtenus comme décrit à l'exemple 1, contenant $1,5 \times 10^9$ spores par gramme de matière sèche dans un réacteur de 1,5 litre ; on ajoute successivement 250 ml d'une solution aqueuse de Tween 80[R] à 0,05 % (p/v) et 1 litre du solvant paraffinique IP 230 OS ; pendant la bioconversion, le milieu réactionnel sera maintenu à 27°C sous agitation et aéré par un système de barbotage d'air à raison de 1 litre par heure.

On ajoute alors 16,6 g (115 mM) d'acide octanoïque et, toutes les huit heures, on ajoute de nouveau de l'acide octanoïque pour remplacer celui qui a été consommé. Après 39 heures, on sépare les grains de sarrasin du milieu réactionnel par filtration sur tamis de maille 0,8 mm et on continue la bioconversion. Après 111 heures, on a transformé 127 g d'acide et on isole par distillation, à 79°C sous 9 kPa, 92 g d'heptanone-2.

On peut aussi effectuer cette opération en introduisant l'acide octanoïque en continu dans le milieu à l'aide d'une pompe ; dans le cas où la concentration en acide est fixée à 70 mM, les résultats sont identiques à ceux de la procédure en discontinu.

Exemple 7 :

On effectue une culture de <u>Penicillium roquefortii</u> sur des grains de sarrasin comme décrit à l'exemple 1. En fin de culture, 30 g de grains sont introduits dans 150 ml d'une solution aqueuse de Tween 80$^R$ à 0,05 % (p/v) et, après quelques minutes d'agitation douce pour détacher les spores externes, le milieu est filtré sur un tamis de maille 0,8 mm et le filtrat est centrifugé à 4 200 tr/min, pour obtenir un culot de quelques millilitres contenant $45 \times 10^9$ spores.

Ce culot séparé est mis en suspension dans 50 ml d'une solution aqueuse de Tween 80$^R$ à 0,05 % et 200 ml du solvant IP 230 OS sont ajoutés ; l'ensemble est placé dans une fiole à 27°C et un léger courant d'air est introduit près de la surface du mélange. On introduit alors 3,3 g (100 mM) d'acide octanoïque et toutes les 12 heures on réajuste à 100 mM la concentration en acide ; après 155 heures, on a transformé 13,8 g d'acide et on peut isoler à partir du milieu réactionnel par distillation 8,8 g d'heptanone-2.

Exemple 8 :

Les spores de <u>Penicillium roquefortii</u> sont obtenues par fermentation solide comme à l'exemple 1, mais sont utilisées en présence des grains de sarrasin ; on introduit alors dans le réacteur $12 \times 10^{11}$ spores, 90 ml d'une solution aqueuse de Tween$^R$ à 0,05 % et 600 ml de solvant IP 230 OS. On introduit de l'acide caproïque dans le milieu, à la concentration initiale de 100 mM, puis, toutes les 12 h, la quantité nécessaire pour ramener la concentration à cette valeur ; le rendement moyen de conversion est de 70 % pour une consommation d'acide de 1,67 mM/h. Au bout de 300 h, on obtient par distillation 51 g/l de nonanone-2.

Exemple 9 :

En opérant dans les mêmes conditions qu'à l'exemple 8 avec de l'acide caproïque en $C_6$, à la concentration de 50 mM, on obtient 17 g/l de pentanone-2 en 250 h.

Exemple 10 :

Les spores d'<u>Aspergillus oryzae</u>, ATCC 1861, sont obtenues à partir de cultures sur milieu gélosé en boîtes de Petri. Le contenu de chaque boîte est mis en suspension dans de l'eau contenant 0,05 % de Tween$^R$ afin d'extraire les spores de la matrice gélosée. On introduit ensuite $3 \times 10^9$ spores en suspension dans 22,5 ml d'eau dans un erlenmeyer contenant 150 ml de solvant paraffinique IP 230 OS et 20 mM d'acide octanoïque. La totalité de l'acide est consommée au bout de 24 heures.

Exemples 11 à 14 :

On effectue la bioconversion de l'acide octanoïque avec d'autres champignons en appliquant le procédé de l'exemple 10. Les résultats figurent dans le tableau 1.

EP 0 390 624 B1

TABLEAU 1

| Espèce | Référence de la souche | Fin de la transformation après |
|---|---|---|
| Penicillium roquefortii | ATCC 6989 | 10 h |
| Penicillium caseicolum | ATCC 123 | 30 h |
| Aspergillus niger | ATCC 9142 | 48 h |
| Trichoderma koningii | DSM 63060 | 30 h |

Exemple comparatif : bioconversion en milieu aqueux

On introduit 115 g de billes de spores immobilisées, comme préparé à l'exemple 1, dans 350 ml d'eau contenant 350 mg/l de chloramphénicol pour éviter les contaminations bactériennes, 0,2 mg/l d'éthanol comme activateur de la bioconversion et du chlorure de calcium à raison de 0,005 mole par litre, pour consolider les billes d'alginate. Le milieu est aéré par soufflage d'air dès la fin de la phase de latence et agité comme à l'exemple 1.

L'air sortant du réacteur est chargé en heptanone-2 qui peut être retenue, au moins partiellement, par barbotage dans du toluène. Après addition de 0,11 g d'acide octanoïque, le pH est ajusté à 6,5 par addition de NaOH aqueux, et maintenu pendant 166 heures à cette valeur par addition d'acide octanoïque. Dans ces conditions, 8 g d'acide sont transformés ; la phase de latence est de 4 heures environ.

## Revendications

1. Procédé d'obtention de méthylcétones aliphatiques en $C_5$ à $C_{10}$ par bioconversion aérobie d'acides gras en $C_6$ à $C_{11}$, par des spores de champignons filamenteux du genre Amastigomycota, caractérisé en ce que le milieu réactionnel est un hydrocarbure aliphatique en $C_8$ à $C_{20}$, ou un mélange de tels hydrocarbures, contenant au plus 20 % d'eau.

2. Procédé selon la revendication 1, caractérisé en ce que le champignon est choisi parmi : Penicillium roquefortii, Penicillium caseicolum, Aspergillus oryzae, Aspergillus niger, Trichoderma koningii.

3. Procédé selon l'une des revendications 1 ou 2, caractérisé en ce que le solvant est un mélange d'hydrocarbures paraffiniques en $C_{13}$ à $C_{16}$.

4. Procédé selon l'une quelconque des revendications 1 à 3, caractérisé en ce que les spores sont libres.

5. Procédé selon l'une quelconque des revendications 1 à 3, caractérisé en ce que les spores sont utilisées dans leur milieu solide de production.

6. Procédé selon l'une quelconque des revendications 1 à 3, caractérisé en ce que les spores sont immobilisées dans une matrice poreuse.

7. Procédé selon l'une quelconque des revendications précédentes, caractérisé en ce que le champignon est Penicillium roquefortii.

8. Procédé selon l'une quelconque des revendications 1 à 7, caractérisé en ce que l'acide gras est l'acide octanoïque.

9. Procédé selon l'une quelconque des revendications 1 à 7, caractérisé en ce que l'acide gras est l'acide caprique.

6

10. Procédé selon l'une quelconque des revendications 1 à 7, caractérisé en ce que l'acide gras est l'acide caproïque.

## Patentansprüche

1. Verfahren zur Herstellung von aliphatischen $C_5$-$C_{10}$-Methylketonen durch aerobe Biokonversion von $C_6$-$C_{11}$-Fettsäuren mittels Sporen von filamentösen Pilzen der Gattung Amastigomycota, dadurch gekennzeichnet, daß es sich beim Reaktionsmedium um einen $C_8$-$C_{20}$-Kohlenwasserstoff oder um ein Gemisch derartiger Kohlenwasserstoffe mit einem Gehalt an höchstens 20 % Wasser handelt.

2. Verfahren nach Anspruch 1, dadurch gekennzeichnet, daß der Pilz ausgewählt ist unter: Penicillium roquefortii, Penicillium caseicolum, Aspergillus oryzae, Aspergillus niger und Trichoderma koningii.

3. Verfahren nach einem der Ansprüche 1 oder 2, dadurch gekennzeichnet, daß es sich beim Lösungsmittel um ein Gemisch aus paraffinischen $C_{13}$-$C_{16}$-Kohlenwasserstoffen handelt.

4. Verfahren nach einem der Ansprüche 1 bis 3, dadurch gekennzeichnet, daß es sich bei den Sporen um freie Sporen handelt.

5. Verfahren nach einem der Ansprüche 1 bis 3, dadurch gekennzeichnet, daß die Sporen in ihrem festen Bildungsmedium verwendet werden.

6. Verfahren nach einem der Ansprüche 1 bis 3, dadurch gekennzeichnet, daß die Sporen in einer porösen Matrix immobilisiert sind.

7. Verfahren nach einem der vorstehenden Ansprüche, dadurch gekennzeichnet, daß es sich bei dem Pilz um Penicillium roquefortii handelt.

8. Verfahren nach einem der Ansprüche 1 bis 7, dadurch gekennzeichnet, daß es sich bei der Fettsäure um Octansäure handelt.

9. Verfahren nach einem der Ansprüche 1 bis 7, dadurch gekennzeichnet, daß es sich bei der Fettsäure um Caprinsäure handelt.

10. Verfahren nach einem der Ansprüche 1 bis 7, dadurch gekennzeichnet, daß es sich bei der Fettsäure um Capronsäure handelt.

## Claims

1. Process for the preparation of $C_5$ to $C_{10}$ aliphatic methyl ketones by the aerobic biotransformation of $C_8$ to $C_{11}$ fatty acids with spores of filamentous fungi of the genus Amastigomycota, characterized in that the reaction medium is a $C_8$ to $C_{20}$ aliphatic hydrocarbon or a mixture of such hydrocarbons containing at most 20% of water.

2. Process according to claim 1, characterized in that the fungus is selected from the group consisting in Penicillium roquefortii, Penicillium caseicolum, Aspergillus oryzae, Asperigillus niger and Trichoderma koningii.

3. Process according to one of claims 1 or 2, characterized in that the solvent is a mixture of $C_{13}$ to $C_{16}$ paraffinic hydrocarbons.

4. Process according to any one of claims 1 to 3, characterized in that the spores are free.

5. Process according to any one of claims 1 to 3, characterized in that the spores are used in their solid production medium.

6. Process according to any one of claims 1 to 3, characterized in that the spores are immobilized in a porous matrix.

7. Process according to any one of the preceding claims, characterized in that the fungus is <u>Penicillium ro-quefortii</u>.

8. Process according to any one of claims 1 to 7, characterized in that the fatty acid is octanoic acid.

9. Process according to any one of claims 1 to 7, characterized in that the fatty acid is capric acid.

10. Process according to any one of claims 1 to 7, characterized in that the fatty acid is caproic acid.